# EUROPÄISCHE PATENTSCHRIFT

(11) **EP 0 516 657 B1**
(45) Veröffentlichungstag und Bekanntmachung des Hinweises auf die Patenterteilung: **10.11.1993**
(21) Anmeldenummer: 91903797.8
(22) Anmeldetag: 22.02.1991
(51) Int. Cl.: A61M 1/36, A61M 1/32

(54) **VERFAHREN UND VORRICHTUNG ZUR BLUTBEHANDLUNG MITTELS SAUERSTOFF UND LICHT**
PROCESS AND DEVICE FOR TREATING BLOOD WITH OXYGEN AND LIGHT
PROCEDE ET DISPOSITIF DE TRAITEMENT DE SANG AU MOYEN D'OXYGENE ET DE LUMIERE

(30) Priorität: 23.02.1990 DE 4005704
(43) Veröffentlichungstag der Anmeldung: 09.12.1992
(73) Patentinhaber: INSTITUT FUR ALTERNATIVE HEILMETHODEN DORIS AMELUNG, 54589 Stadtkyll (DE)
(72) Erfinder: AMELUNG, Hartmut, D-5000 Köln 51 (DE)
(74) Vertreter: Bauer, Wulf, Dr.
(86) Internationale Anmeldenummer: DE9100146
(87) Internationale Veröffentlichungsnummer: WO9112831

(56) Entgegenhaltungen:
- DE-A- 3 500 395
- DE-B- 1 071 291
- DE-U- 8 903 387
- US-A- 4 321 919
- US-A- 4 775 625
- US-A- 4 915 683

## Beschreibung

Die Erfindung bezieht sich auf ein Verfahren zur Blutbehandlung durch Sauerstoff und Licht, bei dem Blut, das sich in einem Reduktionsgefäß befindet, mit Sauerstoff durchströmt und gleichzeitig mit Licht bestrahlt wird, sowie auf eine Vorrichtung zur Durchführung dieses Verfahrens.

Bei dem vorbekannten Verfahren und einer zur Durchführung dieses Verfahrens dienenden Vorrichtung (DE-U 89 03 387) der eingangs genannten Art wird das durch Sauerstoff aufgeschäumte Blut mit energiereicher UV-Strahlung bestrahlt. Verwendet wird ein in das Reduktionsgefäß und in das darin befindliche Blut hineinragender, mit einem lichtdurchlässigen, umhüllenden Gefäß fest verbundener UV-C-Kaltstrahler, dessen Hauptspektrum bei 254 nm liegt. Das Verfahren und die entsprechende Vorrichtung ist als Hämatogene-Oxidations-Therapie, abgekürzt HOT, bekannt, siehe H. Stadtlaender "HOT - Hämatogene-Oxidations-Therapie (photobiologische Oxidationstherapie)", Haug Verlag, 1981 (Schriftenreihe Erfahrungsheilkunde; Band 29).

Bei diesem vorbekannten Verfahren wird einem Patienten zwischen ca. 60 und 100 ml Blut aus der Vene entnommen oder wird eine entsprechende Menge ggfs. konserviertes Blut, das nicht notwendigerweise von dem Patienten stammen muß, verwendet. Diese Blutmenge wird zusammen mit einem Antikoagulans in das Reduktionsgefäß aus Kunststoff oder Glas (insbesondere Einmalgefäß) gegeben, wo es durch Zugabe von reinem Sauerstoff aufgeschäumt und dabei gleichzeitig mit der UV-C-Strahlung bestrahlt wird. Dadurch kommt ein photobiologischer Prozeß in Gang, der im einzelnen näher bei Stadtlaender beschrieben ist. Die so behandelte Blutmenge wird abschließend i.v. oder i.m. in den bzw. einen Patienten injiziert.

Zur Durchführung des Verfahrens wird ein im wesentlichen rotationssymmetrisches Reduktionsgefäß benutzt, das an zwei axial und einander entgegengerichtet verlaufenden Endbereichen einerseits einen Einlaß für das Einspeisen von Sauerstoff und das Einfüllen bzw. die Entnahme von Blut und andererseits eine Öffnung aufweist, durch die die UV-Lichtquelle in das Innere des Reduktionsgefäßes eingeführt werden kann. Bei der vorbekannten Vorrichtung wird ein handelsüblicher UV-Kaltstrahler eingesetzt, der sich in einem länglichen, etwa fingerdicken und vorn abgeschlossenen Quarzröhrchen befindet.

Die bemerkenswerte Erfolgsbilanz der hämatogenen Oxidations-Therapie, verwiesen wird hier zum Nachweis auf die Ausführungen von Stadtlaender, hat dazu geführt, daß Überlegungen angestellt wurden, das Verfahren auch in andere Bereiche auszudehnen und durch geänderte Verfahrensweisen weitere Therapiemöglichkeiten zu erschließen. Dabei wird die Durchperlung des Blutes mit Sauerstoff beibehalten. In Experimenten wurde insbesondere versucht, von der relativ harten UV-Strahlung abzukommen. Weiterhin hat es sich als sehr nachteilig erwiesen, daß das umhüllende Gefäß des UV-Kaltstrahlers für jede neue Anwendung desinfiziert werden muß. Eine sichere Desinfektion ist nicht immer zu erreichen, mangelhafte Desinfektionen führen zu einem erheblichen gesundheitlichen Risiko.

Hier setzt nun die Erfindung ein. Sie hat es sich zur Aufgabe gemacht, das Verfahren und die Vorrichtung der eingangs genannten Art dahingehend weiterzuentwickeln, daß einerseits weniger energiereiche Strahlung eingesetzt und andererseits dem Verfahren ein neuer Anwendungsbereich in der Therapie erschlossen wird.

Ausgehend von dem Verfahren der eingangs genannten Art wird diese Aufgabe dadurch gelöst, daß einfarbiges, vorzugsweise monochromatisches Licht aus dem sichtbaren Spektralbereich zur Bestrahlung des aufgeschäumten Blutes verwendet wird. Vorrichtungsmäßig wird die gestellte Aufgabe gelöst durch die Merkmale des Anspruchs 4.

Es hat sich überraschend herausgestellt, daß durch einfarbiges Licht aus dem sichtbaren Spektralbereich hervorragende Therapieerfolge erzielt werden können. Die Abkehr von dem relativ energiereichen, starke photobiologische und auch photochemische Veränderungen bewirkenden UV-Licht und der Ersatz durch einfarbiges Licht aus dem sichtbaren Spektralbereich mit einer Quantenenergie von etwa nur einem Drittel der UV-Strahlung führt einerseits zu einer gezielteren Variation der Behandlung, da durch Wechsel der Farbfilter unterschiedliche Bereiche aus dem sichtbaren Spektrum (400 bis 800 nm) herausgegriffen werden können und andererseits zu geringeren Nebeneffekten, da die photobiologische Behandlung sanfter und auch mit geringerer Einstrahlung von Wärme erfolgt.

Verfahrensmäßig wird in Abhängigkeit von der jeweiligen Diagnose ausgewählt, mit Licht welcher Farbe, zum Beispiel rot, gelb oder blau, die Einstrahlung erfolgen soll. Das Licht kann monochromatisch oder auch aus einem gewissen Frequenzbereich, beispielsweise 50 bis 80 nm breit, durch entsprechende Filter (Farb- oder Interferenzfilter) aus einem geeigneten Spektrum, im allgemeinen einer Glühlampe (Halogenlampe) herausgefiltert werden. Es können verschiedene Farben nacheinander während der Blutbehandlung eingestrahlt werden, die Abfolge kann vorprogrammiert durch eine mechanische Apparatur gesteuert ablaufen.

Vorrichtungsmäßig erfolgt die Lichterzeugung außerhalb des Reduktionsgefäßes, in Nähe des Eintrittsendes des Lichtleiters befindet sich die Lichtquelle, deren Hauptemission im sichtbaren Bereich liegt. Auf diese Weise können Wärmestrahlung und UV-Anteile außerhalb des Reduktionsgefäßes absorbiert bzw. entfernt werden, so daß sie nicht durch den Lichtleiter zum Blut gelangen können. Schädliche Temperaturerhöhungen und unkontrollierte Bestrahlungen des Blutes außerhalb der gewollten, einfarbigen Lichtbestrahlung werden dadurch vermieden. Weiterhin läßt sich die Lichtmenge, mit der die Bestrahlung des Blutes erfolgt, in weiten Grenzen vorgeben und einregeln.

Vorrichtungsmäßig endet der Lichtleiter entweder in einem austauschbaren, umhüllenden Gefäß, das vorzugsweise als Reagenzglas ausgeführt, oder an einem lichtdurchlässigen Wandbereich des Reduktionsgefäpes, das Licht tritt durch diesen Wandbereich hindurch in das Innere des Reduktionsgefäßes und erreicht so das Blut. Im ersten Fall wird für jede einzelne Behandlung jeweils ein neues, steriles Reagenzglas benutzt. Der Nachteil beim Stand der Technik, daß das dort vorhandene umhüllende Gefäß sterilisiert werden muß, entfällt somit. Das Risiko von Infektionen, insbesondere Übertragung von Aids, durch mangelhafte Sterilisierung ist dadurch beseitigt. Im zweiten Fall ist ein umhüllendes Gefäß gänzlich vermieden. Probleme mit seiner Sterilisierung können daher gar nicht erst auftreten.

Weitere Merkmale und Vorteile der Erfindung ergeben sich aus den Unteransprüchen sowie der nun folgenden Beschreibung von nicht einschränkend zu verstehenden Ausführungsbeispielen, die unter Bezugnahme auf die Zeichnung näher erläutert werden. In dieser zeigen:
- Fig. 1: eine schnittbildliche Ansicht in schematischer Darstellung der erfindungsgemäßen Vorrichtung,
- Fig. 2: eine Ansicht einer Kreisscheibe mit mehreren Farbfiltern und
- Fig. 3: eine Darstellung entsprechend Fig. 1 für eine andere Ausführung.

Zu behandelndes Blut 18 befindet sich in einem Reduktionsgefäß 20, das nur einmal verwendet wird, wodurch eine Sterilisation nach einer Anwendung entfällt, und aus Kunststoff hergestellt ist. Es ist im wesentlichen rotationssymmetrisch. An einem axialen Ende (in der Figur rechts) befindet sich ein Einlaß 22, am anderen axialen Ende eine Öffnung 24. In den Einlaß 22 ist abgedichtet mit einem Stopfen einerseits ein Schlauch 26 für das Einspeisen von Sauerstoff und andererseits ein Anschluß 28 für das Einfüllen und für die Entnahme von Blut eingesetzt. Wie bei der praktischen Durchführung ist das Reduktionsgefäß 20 schräggestellt gezeigt, dabei liegt der Einlaß 22 tief, so daß eingeleiteter Sauerstoff durch das Blut 18 hindurchperlen und sich mit diesem vermischen kann. Der Sauerstoff entweicht über einen dezentralen Auslaß 30, dort ist eine geeignete Sperre dafür vorgesehen, daß keine Flüssigkeit entweichen kann, insbesondere der Überschaum nicht austritt. Hierfür kann, wie bei der vorbekannten Vorrichtung, ein tamponähnlicher, luftdurchlässiger Stopfen verwendet werden, es kann aber auch ein Schlauch angeschlossen werden, der in einem Plastikbeutel unabgedichtet endet.

In die Öffnung 24 ragt ein handelsübliches Reagenzglas 32 hinein, die Öffnung ist so bemessen, daß das Reagenzglas 32 abgedichtet eingeschoben werden kann. Die axiale Länge des Reduktionsgefäßes entspricht im wesentlichen der Länge des Reagenzglases, dies hat zur Folge, daß das Reagenzglas bis in einen trichterförmigen Bereich 34 des Reduktionsgefäßes in Nähe des Einlasses 32 reicht.

In das Reagenzglas 32 ist ein Lichtleiter 36 eingeschoben, der hier als flexibler Lichtleiter aus Fasern ausgeführt ist. Grundsätzlich kann aber auch ein starrer Lichtleiter, beispielsweise ein Kunststoffstab, verwendet werden. Sein Austrittsende 38 befindet sich in Nähe des Bodens des Reagenzglases 32. Am Austrittsende 38 können (in Abweichung von der zeichnerischen Darstellung) auch Zerstreuungsmittel vorgesehen sein, um das austretende Licht in das aufgeschäumte Blut radial einzuleiten. Ohne derartige Mittel erfolgt der Lichtaustritt im wesentlichen axial. Als Zerstreuungsmittel kann beispielsweise eine Zerstreuungs- oder Sammellinse vor das Austrittsende 38 gesetzt werden, in einer anderen Ausführung wird ein verspiegelter Kegel oder ein entsprechend Rotationskörper mit verspiegelter Oberfläche eingesetzt, die das Licht radial nach außen reflektiert.

Im gezeigten Ausführungsbeispiel ist der Lichtleiter 36 verkürzt dargestellt. Sein Eintrittsende 40 befindet sich in einem Gehäuse 42 und wird, wie im folgenden näher beschrieben wird, mit einfarbigem Licht beaufschlagt.

Hierzu befindet sich innerhalb des Gehäuses 42 eine Lichtquelle 44 in Form einer 50 Watt Halogenlampe mit einem W-Glühfaden. Ihr Licht wird durch einen IR-durchlässigen Hohlspiegel im Glühfaden fokussiert und durch eine erste Sammellinse 46, die im Brennpunktabstand angeordnet ist, parallel gebündelt. Hinter der Sammellinse 46 befindet sich ein auswechselbarer Farbfilter 58 und ein IR-Sperrfilter 50 sowie ein UV-Sperrfilter 52.

Anstelle eines Farbfilters 48 können andere geeignete Mittel eingesetzt werden, um aus dem im wesentlichen weißen Licht der Lichtquelle 44 eine Farbe herauszufiltern, beispielsweise Interferenzfilter. Das Farbfilter 48 ist austauschbar angeordnet. Zu der Vorrichtung gehören Farbfilter für unterschiedliche Farben, sie decken im wesentlichen den gesamten sichtbaren Spektralbereich des blau-violetten bis zum dunkelroten Licht ab.

Hinter der Filteranordnung wird das Licht durch eine geeignete Linsenanordnung auf den Ouerschnitt des Lichtleiters 36 gebündelt, es tritt in das Eintrittsende 40 ein und verläßt den Lichtleiter mit möglichst geringem Übertragungsverlust am Austrittsende 38.

In einer geänderten Ausführung kann das Farbfilter 48 auch am Austrittsende 38 vorgesehen sein, beispielsweise kann hier eine gefärbte Linse eingesetzt werden, die zugleich eine Bündelungs- oder Zerstreuungsfunktion übernimmt.

Anstelle einzelner Farbfilter 48 wird vorteilhafterweise ein Satz Farbfilter unterschiedlicher Farben eingesetzt, durch geeignete Hilfsmittel (Zeitgeber und mechanische Betätigungsvorrichtung) werden einzelne Filter in den Strahlengang eingebracht und wieder entnommen. Auf diese Weise läßt sich die Behandlung von Blut 18 in dem Reduktionsgefäß 20 automatisieren, es kann beispielsweise drei Minuten mit roten Licht, eine Minute mit gelbem Licht, eine Minute mit blauem Licht usw. bestrahlt werden, ohne daß manuelle Handgriffe nötig sind, umd die Behandlungsdauer zu überwachen und das Behandlungslicht vorzugeben.

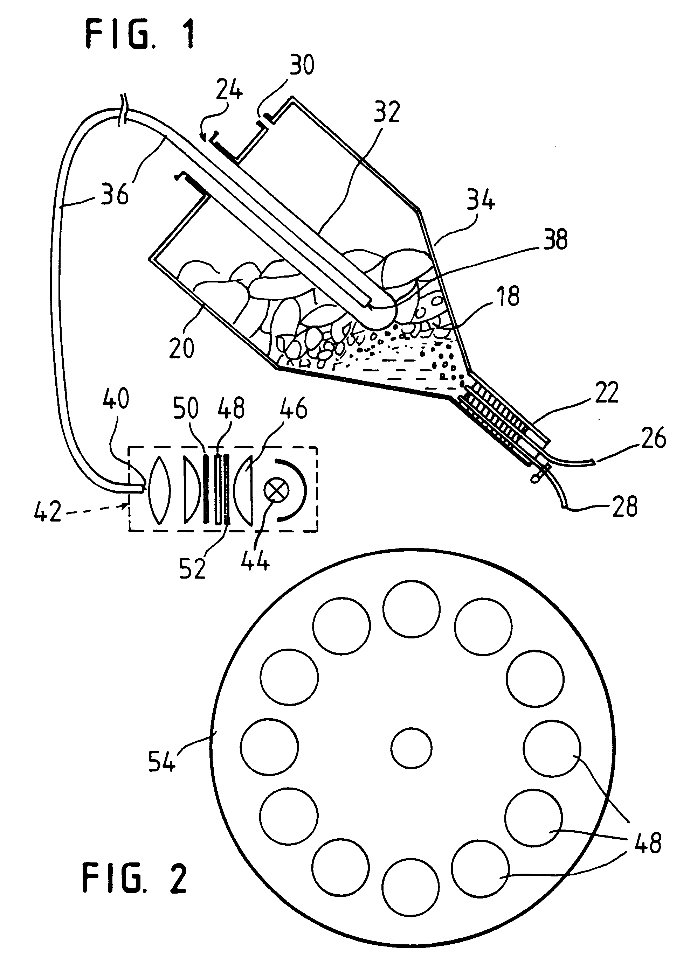

Im Ausführungsbeispiel nach Figur 2 ist hierzu eine Kreisscheibe 54 gezeigt, in die einzelne Farbfilter 48 unterschiedlicher Färbung eingesetzt sind. Durch Drehen der Kreisscheibe 54 um eine durch ihren Mittelpunkt gehende Achse können die einzelnen Farbfilter 48 in den Strahlengang eingeschwenkt werden. Dies kann manuell erfolgen, indem der Außenrand der Kreisscheibe entsprechend markiert und gerändelt ist, es kann aber auch motorisch ablaufen.

Bei der praktischen Durchführung befindet sich innerhalb des Reduktionsgefäpes ca. 60 bis 100 ml Blut. Durch Einleiten von insgesamt ca. 100 ml Sauerstoff, der langsam zugeführt wird und durch die Blutmenge hindurchperlt, schäumt das Blut auf und bekommt eine hellere Farbe. Gleichzeitig wird das Blut mit einfarbigem Licht beleuchtet, das aus dem Kaltlichtleiter 36 und durch das abdichtende Reagenzglas 32 hindurch in die Blumenge 18 gelangen kann. Die Gesamtbehandlungsdauer (O₂-Einleiten und Bestrahlung mit Licht) dauert einige Minuten, beispielsweise zehn bis zwanzig Minuten. Die Bestrahlung mit Licht kann in einzelne Zeitabschnitte unterteilt werden, in diesen Zeitabschnitten werden unterschiedliche Lichtfarben gleichzeitig oder nacheinander eingestrahlt.

Anstelle der im Ausführungsbeispiel besprochenen Halogenlampe können auch andere Lichtquellen eingesetzt werden, beispielsweise Leuchtdioden, die ja von Hause aus schon einfarbiges Licht emittieren und für unterschiedliche Emissionsfarben angeboten werden, Laser im sichtbaren Spektralbereich, beispielsweise durchstimmbare Farbstofflaser, Spektrallampen und dergleichen.

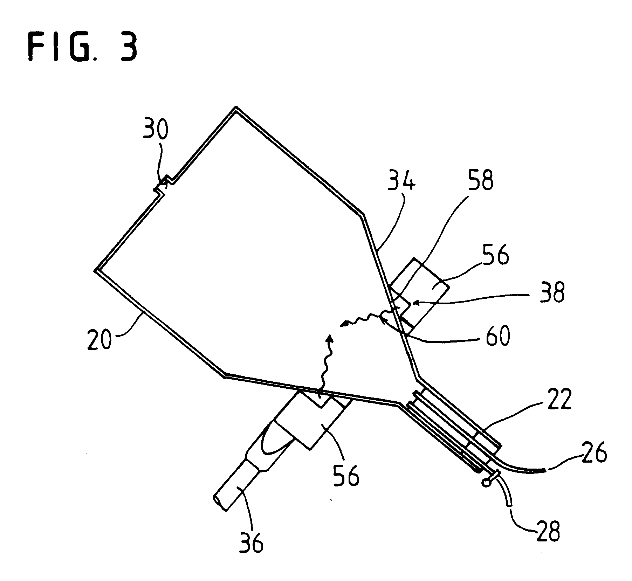

Im Ausführungsbeispiel nach Fig. 3 ist das Reduktionsgefäß 20 aus glasklarem Kunststoff gefertigt. Das Licht wird bei dieser Ausführung nicht mehr durch ein separates Reagenzglas 32, sondern unmittelbar durch die Außenwand des Reduktionsgefäßes dem Blut zugeführt. Hierzu ist das Austrittsende 38 des flexiblen Lichtleiters 36 als Glasfaser-Ringlicht 56 ausgebildet. Ringlichter haben einen kegelförmigen Lichtaustritt. Ein geeignetes Glasfaser-Ringlicht wird beispielsweise durch die schweizerische Firma Volpi AG, CH-8952 Schlieren/Zürich angeboten. In der Ausführung nach Fig. 3 ist der ringförmige Körper des Glasfaser-Ringlichtes 58 über den Einlaß 22 hinweg auf den trichterförmigen Bereich 34 geschoben, wo er anliegt. Ein ringförmiger Teilbereich dieses trichterförmigen Bereichs 34 bildet einen Wandbereich 58, durch den das ringförmig abgestrahlte Licht in das Innere des Reduktionsgefäßes 20 eintritt und damit vom Blut aufgenommen werden kann. Im gezeigten Ausführungsbeispiel sind die einfarbigen Lichtstrahlen durch gewellte Pfeile 60 symbolisiert. Sie verlaufen im wesentlichen rechtwinklig zum Wandbereich 58, so daß möglichst geringe Reflexionen auftreten.

Die Ausführung nach Fig. 3 hat den Vorteil, daß kein separates Reagenzglas 32, das wie das Reduktionsgefäß 20 nur einmal verwendet werden kann, benötigt wird. Dementsprechend ist die Öffnung 24, die beim Ausführungsbeispiel nach Fig. 1 vorgesehen ist, nicht notwendig. In der Ausführung nach Fig. 3 ist der Auslaß 30 für das Ausströmen von Luft und Sauerstoff axial angeordnet, was die Herstellung des Reduktionsgefäßes 20 vereinfacht.

Unter dem Begriff Lichtemitter wird aktive oder passive Lichtabstrahlung verstanden, Lichtemitter in diesem Sinne ist somit z.B. die Austrittsfläche eines Lichtleiters, die aktive Fläche einer Leuchtdiode oder eine Glühlampe.

## Patentansprüche

1. Verfahren zur Blutbehandlung durch Sauerstoff und Licht, bei dem Blut, das sich in einem Reduktionsgefäß (20) befindet, mit Sauerstoff durchströmt und gleichzeitig mit Licht bestrahlt wird, dadurch gekennzeichnet, daß einfarbiges, vorzugsweise monochromatisches Licht aus dem sichtbaren Spektralbereich verwendet wird.

2. Verfahren nach Anspruch 1, dadurch gekennzeichnet, daß der IR-und/oder der UV-Anteil des Bestrahlungslichtes durch spezielle Maßnahmen entfernt wird.

3. Verfahren nach Anspruch 1 oder 2, dadurch gekennzeichnet, daß das einfarbige Licht durch einen Wandbereich (58) des Reduktionsgefäßes (20) hindurch in das innere des Reduktionsgefäßes (20) eintritt.

4. Vorrichtung zur Blutbehandlung durch Sauerstoff und durch Licht zur Durchführung des Verfahrens nach Anspruch 1, mit einem Reduktionsgefäß (20), das einen Einlaß (22) und einen Auslaß (30) aufweist, wobei am Einlaß (22) einerseits ein Schlauch (26) für das Einspeisen von Sauerstoff und andererseits ein Anschluß (28) für das Einfüllen und für die Entnahme von Blut abgedichtet angeordnet ist und durch den Auslaß (30) Gas entweichen kann,
dadurch gekennzeichnet, daß ein Lichtleiter (26) vorgesehen ist, dessen Austrittsende (38) entweder durch eine Öffnung (24) in das Reduktionsgefäß (20) ragt und sich in einem lichtdurchlässigen, wasserdichten, umhüllenden Gefäß (Reagenzglas 32) befindet, das lösbar mit dem Reduktionsgefäß (20) und dem Lichtleiter (26) verbunden ist oder sich an einem lichtdurchlässigen Wandbereich des Reduktionsgefäßes (20) befindet und in das Innere des Reduktionsgefäßes (20) gerichtet ist, daß dem Lichtleiter (26) eingangsseitig eine sichtbares Licht abstrahlende Lichtquelle (44) zugeordnet ist, und daß zwischen dieser Lichtquelle (44) und dem umhüllenden Gefäß (Reagenzglas 32) bzw. dem lichtdurchlässigen Wandbereich (58) eine Vorrichtung zur Bildung von einfarbigen, insbesondere monochromatischem Licht, vorzgusweise ein Farbfilter (48) austauschbar angeordnet ist.

5. Vorrichtung nach Anspruch 4, dadurch gekennzeichnet, daß die Vorrichtung zur Bildung von einfarbigem Licht, vorzugsweise das Farbfilter (48) zwischen der Lichtquelle (44) und einem Eintrittsende (40) des Lichtleiters (36) angeordnet ist.

6. Vorrichtung nach Anspruch 4, dadurch gekennzeichnet, daß im Lichtweg ein Sperr- oder Absorptionsfilter für IR- und/oder UV-Licht vorgesehen ist.

7. Vorrichtung nach Anspruch 4, dadurch gekennzeichnet, daß eine Vorrichtung zur Zerstreuung des Lichtes am Austrittsende (38), insbesondere eine Linse (Sammel- oder Zerstreuungslinse) oder ein kegelartiger Rotationskörper mit reflektierender Oberfläche angeordnet ist.

8. Vorrichtung nach Anspruch 4, dadurch gekennzeichnet, daß sich die Lichtquelle (44) und das Eintrittsende (40) des Lichtleiters (36) in einem Gehäuse (42) befinden und daß diesem Gehäuse eine Zeitschaltuhr sowie eine mechanische Betätigung für das Ein- und Ausschalten sowie das Einbringen verschiedener Farbfilter (48) zugeordnet ist.

9. Vorrichtung nach Anspruch 4, dadurch gekennzeichnet, daß das Reduktionsgefäß (20) einen kegelförmig verlaufenden Bereich aufweist, in dem sich der Wandbereich (58) befindet, und daß am Austrittsende des Lichtleiters ein Glasfaser-Ringlicht (56) angeordnet ist, das am Wandbereich (58) anliegt.

## Claims

1. Method for treating blood with oxygen and light, in which method the blood, which is in a reduction container (29), is penetrated by oxygen and at the same time irradiated with light, characterised by the use of single-color-light, especially monochromatic light, of the visible optical spectrum.

2. Method according to claim 1, characterised in that the infrared portion and/or the ultraviolet portion of the irradiaring light is removed by special means.

3. Method according to claim 1 or 2, characterised in that the single-color-light enters the reduction container (20) through a wall section (58) thereof.

4. Device for treating blood with oxygen and light to perform the method of claim 1, with a reduction container (20), having an inlet (22) and an outlet (30), whereby the inlet (22) is equipped with on one hand a flexible tube attached thereto for injecting the oxygen and on the other hand with an attachment (28) for the charging and discharging of blood sealingly attached thereto and the outlet (30) being adapted for an outflow of gas, characterised in that an optic light guide (26) is provided, the outlet end (38) thereof either extends through an opening (24) in the reduction container (20) and is positioned in a translucent, water-light, covering vessel (test tube 32), which is detachably fixed to the reduction container (20) and to the optic light guide (26) or is arranged at a translucent wall portion of the reduction container (20) and points towards the inner space of the reduction container (20), that a light source (44) delivering visible light is arranged to the input portion of the optic light guide (26), and that a means for producing single-color-light, especially monochromatic light, is detachably arranged between the light source (44) and the covering vessel (test tube 32) or the translucent wall portion (58), respectively, which means for producing single-color-light is preferably a color-filter (48).

5. Device according to claim 4, characterised in that the means for producing single-color-light, preferably monochromatic light, is arranged between the light source (44) and an input end (40) of the optic light guide (36).

6. Device according to claim 4, characterised in that a rejection filter or and absorption filter for infrared and/or ultraviolet light is arranged in the optical light path.

7. Device according to claim 4, characterised in that means for dispersion of light are arranged at the outlet end (38), especially a lens (collimating or dispersing lens) or a cone-like body of revolution having a reflecting surface.

8. Device according to claim 4, characterised in that the light source (44) and the inlet end (40) of the optic light guide (36) are in a housing (42) and in that a time switch, and a mechanical means for switching on and off and for inserting different color filters (48) is attached to said housing.

9. Device according to claim 4, characterised in that the reduction container (20) exhibits a conically extending portion, in which the said wall portion (58) is located, and in that a fiber concentric light arrangement (56) is arranged at the outlet end of the optic light guide and is attached to the said wall portion (58).

## Revendications

1. Procédé de traitement du sang à l'oxygène et à la lumière, dans lequel le sang qui se trouve dans un récipient de reduction (20) est traversé par un courant d'oxygène et exposé en même temps à la lumière, caractérisé en ce qu'on utilise de préférence de la lumière monocouleur en particulier de la lumière monochromatique du domaine spectral visible.

2. Procédé selon la revendication 1, caractérisé en ce que la fraction IR et/ou la fraction UV de la lumière d'irradiation est supprimée par des moyens particuliers.

3. Procédé selon la revendication 1 ou 2, caractérisé en ce que la lumière monochromatique pénètre à l'intérieur du récipient de réduction (20) à travers une zone (58) de la paroi du récipient de réduction (20).

4. Dispositif de traitement du sang à l'oxygène et à la lumière pour la mise en oeuvre du procédé selon la revendication 1, comportant un récipient de réduction (20) qui présente une entrée (22) et une sortie (30), à l'entrée (22) étant prévus, de manière étanche, d'une part, un tuyau (26) pour l'alimentation en oxygène et, d'autre part, un raccord (28) pour l'arrivée et le prélèvement du sang et du gaz pouvant s'echapper par la sortie (30), caractérisé en ce qu'il est prévu un guide de lumière (26) dont l'extrémité de sortie (38) s'engage par un orifice (24) dans le récipient de réduction (20) et se trouve dans un récipient d'enveloppe translucide, étanche à l'eau (tube à réaction 32), qui est relié de manière amovible avec le récipient de reductlon (20) et le guide de lumière (26), ou se trouve sur une zone translucide de la paroi du récipient de réduction (20) et est orientée vers l'intérieur du récipient de réduction (20), en ce qu'au guide de lumière (26) est associée, côté entrée, une source lumineuse (44) émettant une lumière visible et en ce qu'entre cette source lumineuse (44) et le recipient d'enveloppe (tube à réaction 32) ou la zone (58) translucide de la paroi, il est prévu un dispositif interchangeable, de préférence un filtre coloré (48) en vue de la formation d'une lumière d'une seule couleur, en particulier monochromatique.

5. Dispositif selon la revendication 4, caractérisé en ce que le dispositif de formation d'une lumière d'une seule couleur, de préférence le filtre coloré (48), est prévu entre la source lumineuse (44) et une extrémité d'entrée (40) du guide de lumière (36).

6. Dispositif selon la revendication 4, caractérisé en ce qu'il est prévu un filtre d'arrêt ou un filtre d'absorption pour lumière IR et/ou lumière UV.

7. Dispositif selon la revendication 4, caractérisé en ce qu'il est prévu un dispositif de dispersion de la lumière à l'extrémité de sortie (38), en particulier une lentille (lentille de convergence ou lentille de dispersion) ou un corps de rotation, à la manière d'un cône, avec surface réfléchissante.

8. Dispositif selon la revendication 4, caractérisé en ce que la source lumineuse (44) et l'extrémité d'entrée (40) du guide de lumière (36) se trouvent dans un boîtier (42) et en ce qu'à ce boltier sont associées une minuterie ainsi qu'une commande mécanique pour la mise en marche et l'arret ainsi que la mise en place de différents filtres colorés (48).

9. Dispositif selon la revendication 4, caractérisé en ce que le récipient de réduction (20) présente une zone de forme conique dans laquelle se trouve la zone de paroi (58) et en ce qu'à l'extrémité de sortie du guide de lumière est prévu un anneau de lumière en fibres de verre (56) qui s'applique contre la zone de paroi (58).
